# EUROPÄISCHE PATENTANMELDUNG

(11) **EP 4 205 635 A1**
(43) Veröffentlichungstag der Anmeldung: **05.07.2023**
(21) Anmeldenummer: 21218288.5
(22) Anmeldetag: 30.12.2021
(51) Int. Cl.: A61B 5/00, A61B 5/0531

(54) **WUNDAUFLAGE MIT BEHANDLUNGS- UND SENSORELEMENT**

(71) Anmelder: Paul Hartmann AG, 89522 Heidenheim (DE)
(72) Erfinder: DEIBLER, Martin, 89542 Herbrechtingen (DE); CROIZAT, Pierre, 89542 Herbrechtingen (DE)

(57) **Zusammenfassung**

Die Erfindung betrifft eine Wundauflage zur Behandlung einer Wunde. Insbesondere betrifft die Erfindung eine Wundauflage zur Behandlung und Überwachung einer Wunde. Die erfindungsgemäße Wundauflage vermag in einem örtlich beschränkten Bereich einer Wundstelle einen therapeutischen Effekt auszuüben. Die Wundauflage umfasst eine Abdeckschicht, ein Behandlungselement sowie ein Sensorelement. Das Behandlungselement umfasst mindestens zwei unabhängig aktivierbare Einheiten und das Sensorelement umfasst mindestens zwei unabhängig aktivierbare Einheiten. Die Wundauflage umfasst mindestens zwei identische Anordnungen, welche jeweils mindestens eine unabhängig aktivierbare Einheit des Sensorelements und mindestens eine unabhängig aktivierbare Einheit des Behandlungselements umfassen. Die unabhängig aktivierbaren Einheiten des Sensorelements und die unabhängig aktivierbaren Einheiten des Behandlungselements sind so angeordnet, dass durch wiederholtes, lückenloses und auf translationssymmetrische Operationen basierendes Aneinanderfügen der identischen Anordnungen ein im Raum periodisches Muster gebildet wird. Die regelmäßige Anordnung der Einheiten des Sensorelements erlaubt eine verbesserte Aufnahme der die Wunde betreffenden Daten und erleichtert die Auswertung der Wundeigenschaften.

## Beschreibung

Die Erfindung betrifft eine Wundauflage zur Behandlung einer Wunde. Insbesondere betrifft die Erfindung eine Wundauflage zur Behandlung und Überwachung einer Wunde.

Wundauflagen zur Behandlung von Wunden sind bekannt. Dabei wird eine Wundauflage auf eine Wunde zur Wundbehandlung aufgebracht, um diese unter anderem vor äußeren Einflüssen wie Verunreinigungen zu schützen und ein wundheilungsförderndes Milieu zu schaffen.

Eine Überprüfung des Heilungsprozesses der Wunde wird in der Regel durch eine Inspektion der Wunde durch einen Wundspezialisten, beispielsweise durch einen Arzt oder Krankenpfleger, durchgeführt. Dazu muss die Wundauflage in regelmäßigen Intervallen von der Wunde entfernt werden und von dem Wundspezialisten bewertet werden. Dadurch kann die Wundruhe gestört werden und somit der Wundheilungsprozess negativ beeinflusst werden.

Es kann notwendig sein, dass ein Patient mit der Wunde den Wundspezialisten mehrfach aufsuchen muss, was für den Patienten belastend ist. Auch werden durch die regelmäßigen Bewertungen durch den Wundspezialisten Ressourcen des Wundspezialisten gebunden.

Eine Quantifizierung und eine objektive Bewertung des Wundheilungsprozesses ist auch durch den Wundspezialisten kaum möglich. Auch eine Bewertung der Effektivität des Therapieansatzes ist nur sehr eingeschränkt möglich.

In der Wunde kann sich ein Biofilm bilden. Biofilme können eine große Herausforderung im Bereich von Wunden darstellen und die Wundheilung beeinträchtigen.

Der Biofilm kann mechanisch entfernt werden. Dazu muss die Wundauflage von der Wunde entfernt werden und von einem Wundspezialisten behandelt werden.

Auch kann zur Förderung des Heilungsprozesses gewünscht sein, therapeutisch wirksame Substanzen in die Wunde abzugeben, beispielsweise antimikrobielle Substanzen und Medikamente.

Der Erfindung liegt die Aufgabe zu Grunde, eine Wundauflage bereitzustellen, die eine verbesserte Behandlung einer Wunde ermöglicht. Eine weitere Aufgabe liegt darin, eine Wundauflage bereitzustellen, durch die eine ressourcenschonende Behandlung einer Wunde ermöglicht wird. Eine weitere Aufgabe der Erfindung liegt darin, eine Wundauflage bereitzustellen, durch die eine präzise Behandlung derjenigen Bereiche der Wunde, welche tatsächlich einer Behandlung bedürfen, ermöglicht wird. Eine weitere Aufgabe liegt darin, eine Wundauflage bereitzustellen, durch die eine durchgehende Überwachung eines Wundheilungsprozesses ermöglicht wird. Eine weitere Aufgabe liegt darin, eine Wundauflage bereitzustellen, durch die eine objektive Bewertung eines Wundheilungsprozesses ermöglicht wird.

Die Aufgaben werden gelöst durch eine Wundauflage nach Anspruch 1, durch ein Auswertungsmittel nach Anspruch 10 und durch ein Verfahren nach Anspruch 13. Die erfindungsgemäße Wundauflage vermag in einem örtlich beschränkten Bereich einer Wundstelle einen therapeutischen Effekt auszuüben. Die Wundauflage umfasst eine Abdeckschicht, ein Behandlungselement sowie ein Sensorelement. Sie kann weiterhin eine optionale Wundkontaktschicht, die bei Gebrauch der Wundauflage wundseitig angeordnet ist, umfassen.

Das Behandlungselement umfasst mindestens zwei unabhängig aktivierbare Einheiten und das Sensorelement umfasst mindestens zwei unabhängig aktivierbare Einheiten. Die Wundauflage umfasst mindestens zwei identische Anordnungen, welche jeweils mindestens eine unabhängig aktivierbare Einheit des Sensorelements und mindestens eine unabhängig aktivierbare Einheit des Behandlungselements umfassen. Die unabhängig aktivierbaren Einheiten des Sensorelements und die unabhängig aktivierbaren Einheiten des Behandlungselements sind so angeordnet, dass durch wiederholtes, lückenloses und auf translationssymmetrische Operationen basierendes Aneinanderfügen der identischen Anordnungen ein im Raum periodisches Muster gebildet wird.

Die regelmäßige Anordnung der Einheiten des Sensorelements erlaubt eine verbesserte Aufnahme der die Wunde betreffenden Daten und erleichtert die Auswertung der Wundeigenschaften. Ebenfalls kann durch die Regelmäßigkeit der Anordnungen der Einheiten des Sensorelements die Analyse der Daten vereinfacht und beschleunigt werden, da keine beliebigen Abstände zwischen den Ortkoordinaten, die einzelnen Datenpunkten zugeordnet sind, berücksichtigt werden müssen.
Ähnliche Vorteile lassen sich auch für die Einheiten des Behandlungselements beobachten. Die Einheiten des Behandlungselements können einzeln und unabhängig voneinander aktiviert werden. Damit ist eine gezielte Behandlung der Wunde möglich, ohne dass gesundes Gewebe durch überflüssige Behandlung in Mitleidenschaft gezogen werden muss. Die regelmäßige Anordnung der Einheiten des Behandlungselements stellt sicher, dass alle Bereiche der Wunde durch die aktivierten Einheiten des Behandlungselements erreicht werden, ohne, dass z.B. eine Erhöhung der Intensität der Behandlung dafür notwendig ist. So ist eine schonendere Behandlung der Wunde sichergestellt. Die Kombination dieser beiden Vorteile ermöglicht eine adaptive Wundbehandlung über die Zeit. Verringert sich beispielsweise die Wundgröße, so kann die Behandlung durch eine Reduktion der aktivierten Einheiten des Behandlungselements angepasst werden.

Die Wundauflage umfasst mindestes zwei Einheiten des Behandlungselements und mindestens zwei Einheiten des Sensorelements, bevorzugt mindestes fünf Einheiten des Behandlungselements und mindestens fünf Einheiten des Sensorelements, besonders bevorzugt mindestens 10 Einheiten des Behandlungselements und mindestens 10 Einheiten des Sensorelements, insbesondere mindestens 20 Einheiten des Sensorelements und mindestens 20 Einheiten des Behandlungselements.

Da die in der Wundauflage vorhandenen Einheiten des Behandlungselements selektiv über einem ausgewählten Bereich der Wundfläche aktiviert werden können, ist eine gezielte Behandlung der Wunde möglich, ohne dass unversehrtes Gewebe in der Umgebung der Wunde beeinträchtigt wird. Die Sensoreinheit der Wundauflage macht eine durchgehende Überwachung des Wundzustandes möglich. Die Kombination von Sensoreinheit und Behandlungseinheit erlaubt eine Behandlung und Inspektion der Wunde über einen längeren Zeitraum, ohne dass die Wundauflage entfernt werden muss. Die Wundauflage kann beispielsweise mindestens 3 Tage, bevorzugt mindestens 7 Tage, besonders bevorzugt mindestens 14 Tage ohne Verbandwechsel auf der Wunde verbleiben.

Allgemein kann eine Wundauflage eine Abdeckung des Wundbereichs sein, durch die das Eindringen von Fremdkörpern in die Wunde reduziert oder vermieden wird. Die Fremdkörper können Staub, Schmutz oder sonstige Verunreinigungen sein. Die Wundauflage kann ausgebildet sein Blut, Wundexsudat, Mikroorganismen und/oder Biofilm-Fragmente aufzunehmen. Die Wundauflage kann vorzugsweise eine Wundauflage zur feuchten Wundbehandlung sein.

Bevorzugt ist die Wundauflage eine selbsthaftende Wundauflage. Eine selbsthaftende Wundauflage kann auf einen Hautabschnitt aufgeklebt werden, um an dem Hautabschnitt lösbar zu haften. Alternativ kann die Wundauflage nicht selbsthaftend sein, wobei dann zusätzliche Fixierungsmittel, wie z.B. Klebestreifen, erforderlich sind.

Die Wundauflage kann insgesamt flexibel oder nachgiebig ausgestaltet sein. Dadurch kann die Wundauflage auf einen unebenen Hautabschnitt aufbringbar sein. Auch kann die Wundauflage eine Bewegung des Hautabschnitts ermöglichen, während die Wundauflage auf den Hautabschnitt aufgebracht ist. Beispielsweise kann die Wunde an einem Knie vorliegen. Dazu muss die Wundauflage an der unebenen Haut anliegen können. Auch ist es vorteilhaft, wenn das Körperteil (z.B. das Knie) beweglich bleibt, ohne dass die Wundauflage ihre Haftung zu dem Hautabschnitt des Körperteils verliert. Alternativ kann die Wundauflage unflexibel oder unnachgiebig, beispielsweise starr, sein. Eine starre Wundauflage kann einen zusätzlichen Schutz der Wunde vor einem Eindringen eines Fremdkörpers bereitstellen.

Eine Wunde kann ein Defekt von Deckgewebe der menschlichen Haut sein. Der Defekt kann einen Gewebeverlust umfassen oder keinen Gewebeverlust umfassen.

Der Hautabschnitt, auf den die Wundauflage aufbringbar ist, also die Wundstelle, umfasst die Wunde. Es ist bevorzugt, dass die Wundauflage auf einen Teil des Hautabschnitts außerhalb der Wunde (auch als intakte Haut bezeichnet) und auf die Wunde aufbringbar ist. Die Wundauflage kann daher eine größere Fläche als die Wunde aufweisen. Die Wunde kann vollständig von der Wundauflage bedeckbar sein.

Die Wundauflage kann eine Wundkontaktschicht umfassen. Die Wundkontaktschicht kann bei Gebrauch der Wundauflage den Hautabschnitt kontaktieren, während die Wundauflage auf den Hautabschnitt aufgebracht ist. Die Wundkontaktschicht kann bei Gebrauch der Wundauflage gleichfalls die Wundoberfläche kontaktieren

Die Wundauflage kann eine Zwischenschicht umfassen. Die Zwischenschicht kann ein absorbierendes Material aufweisen. Alternativ oder zusätzlich kann die Zwischenschicht ein bioaktives Material umfassen. Die Zwischenschicht kann zwischen der Abdeckschicht und der Schicht, die zumindest einige der Einheiten des Sensor- und/oder Behandlungselements bedeckt oder aufnimmt, angeordnet sein. Die Zwischenschicht kann als Absorptionsschicht oder als Transferschicht ausgeführt sein. Die Zwischenschicht kann ein Fasermaterial, Fleece, ein Vlies, ein Mikrofasermaterial und/oder einen Superabsorber (insbesondere ein AcrylatPolymer) umfassen.

Die Wundauflage umfasst eine Abdeckschicht. Die Abdeckschicht kann die Wundauflage zumindest teilweise bedecken. Bevorzugt bedeckt die Abdeckschicht die Wundauflage vollständig. Die Abdeckschicht kann auf der bei Gebrauch der Wundauflage wundabseitig vorhandenen Seite der Zwischenschicht angeordnet sein. Die Abdeckschicht kann elastisch ausgebildet sein. In einer Ausführungsform ist die Abdeckschicht lösbar mit der Wundauflage verbunden. Die Abdeckschicht kann als Komponente separat von den anderen Komponenten der Wundauflage zur Verfügung gestellt werden. In einer anderen Ausführungsform wird die Abdeckschicht während des Herstellungsprozesses unlösbar in die Wundauflage integriert.

Die Anordnungen aus einer Untermenge der unabhängig aktivierbaren Einheiten des Sensorelements und aus einer Untermenge der unabhängig aktivierbaren Einheiten des Behandlungselements wird mindestens einmal wiederholt, das heißt jede Anordnung ist baugleich. Es können beispielsweise 2 bis 5000 Wiederholungen sein, bevorzugt sind 4 bis 1000 Wiederholungen der Anordnung, besonders bevorzugt sind 12 bis 1000 Wiederholungen der Anordnung, insbesondere 100 bis 500 Wiederholungen der Anordnung.

Die mindestens zwei unabhängig aktivierbaren Einheiten des Sensorelements umfassen jeweils mindestens einen der folgenden Bauteile: Photosensoren, piezoelektrische Ultraschallsensoren, Impedanzsensoren oder Widerstandssensoren. Die mindestens zwei unabhängig aktivierbaren Einheiten des Behandlungselements umfassen jeweils mindestens einen der folgenden Bauteile: Photodioden, Lichtleiter, piezoelektrische Ultraschallemitter, elektrisch leitende Elemente oder Thermoelemente. In einer Ausführungsform der Anordnung kann ein Bauteil mehrmals vorkommen. Jedoch werden erfindungsgemäß mindestens ein Bauteil, das zur Behandlung ausgelegt ist, und mindestens ein Bauteil, das als Sensor wirkt, in einer Anordnung kombiniert. Beispielsweise wäre eine Anordnung aus einem piezoelektrischen Ultraschallsensor, wobei der Ultraschallsensor eine Einheit des Sensorelements ist, und einem piezoelektrischen Ultraschallemitter, wobei der piezoelektrische Ultraschallemitter eine Einheit des Behandlungselements ist, möglich. Eine Anordnung aus zwei Photosensoren in Kombination mit einer Photodiode, sowie einem piezoelektrischen Ultraschallemitter sind denkbar.

Es können beispielsweise 2 bis 5000 Wiederholungen der Anordnung verwendet werden, wobei die Anordnungen in diesem Beispiel je einen piezoelektrische Ultraschallsensor als Einheit des Sensorelements und einen piezoelektrischen Ultraschallemitter als Einheit des Behandlungselements umfasst. Wird beispielsweise eine Anordnung aus 4 Fotosensoren als Einheiten des Sensorelements und aus einer Fotodiode als Einheit des Behandlungselements verwendet, so sind 400 Wiederholungen der Anordnung möglich.

Das Volumen, das jede der Anordnungen aus einer Untermenge der unabhängig aktivierbaren Einheiten des Sensorelements und aus einer Untermenge der unabhängig aktivierbaren Einheiten des Behandlungselements einnimmt, ist ein Parallelepiped in drei Dimensionen oder ein Parallelogramm, falls alle Einheiten des Sensorelements und alle Einheiten des Behandlungselements in einer Ebene angeordnet sind. Eine Verschiebung einer Anordnung entlang einer ganzzahligen Linearkombination der Basisvektoren, die das Parallelepiped bzw. das Parallelogramm aufspannen, ist eine translationssymmetrische Operation. Die Basisvektoren sind linear unabhängig.

Die Anordnungen aus einer Untermenge der unabhängig aktivierbaren Einheiten des Sensorelements und aus einer Untermenge der unabhängig aktivierbaren Einheiten des Behandlungselements werden wiederholt, und lückenlos durch translationssymmetrische Operationen aneinandergefügt.

Die mindestens zwei unabhängig aktivierbaren Einheiten des Sensorelements können bevorzugt in einer Ebene aus einer Menge erster Ebenen, welche parallel zu der Wundoberfläche sind, angeordnet sein, und die mindestens zwei unabhängig aktivierbaren Einheiten des Behandlungselements in einer Ebene aus einer Menge zweiter Ebenen, welche parallel zu der Wundoberfläche sind, angeordnet sein.

In einer bevorzugten Ausführungsform werden die unabhängig aktivierbaren Einheiten des Sensorelements in einer Ebene aus einer Menge erster Ebenen angeordnet, wobei die Menge erster Ebenen nur eine einzige Ebene beinhaltet, und die unabhängig aktivierbaren Einheiten des Behandlungselements in einer Ebene aus einer Menge zweiter Ebenen angeordnet, wobei die Menge zweiter Ebenen nur eine einzige Ebene beinhaltet. Die unabhängig aktivierbaren Einheiten des Sensorelements und die unabhängig aktivierbaren Einheiten des Behandlungselements sind bevorzugt versetzt angeordnet, das heißt, dass sich die Projektionen der unabhängig aktivierbaren Einheiten des Sensorelements und die Projektionen der unabhängig aktivierbaren Einheiten des Behandlungselements orthogonal auf die Wundflache nicht überschneiden.

In einer bevorzugten Ausführungsform können die Menge erster Ebenen mit der Menge zweiter Ebenen identisch sein.
Wird nur eine einzige Ebene verwendet, so ist die Fläche, den jede der Anordnungen aus einer Untermenge der unabhängig aktivierbaren Einheiten des Sensorelements und aus einer Untermenge der unabhängig aktivierbaren Einheiten des Behandlungselements einnimmt, ein Parallelogramm.

Zwischen zwei benachbarten Einheiten der mindestens zwei unabhängig aktivierbaren Einheiten des Sensorelements kann ein Abstand zwischen 0,2 mm und 10,0 mm, bevorzugt zwischen 0,5 mm und 9,0 mm, besonders bevorzugt zwischen 1,0 mm und 8,0 mm vorhanden sein. Nach einer weiteren Ausführungsform beträgt der Abstand zwischen 2,0 mm und 7,0 mm, bevorzugt zwischen 3,0 mm und 7,0 mm, besonders bevorzugt 5,0 mm. Der Abstand von zwei benachbarten Einheiten der mindestens zwei unabhängig aktivierbaren Einheiten des Sensorelements kann in einer anderen bevorzugten Ausführungsform zwischen 50 % und 150 % des maximalen Durchmessers einer unabhängig aktivierbaren Einheit des Sensorelements betragen.

Der vorstehend aufgeführte Abstand kann jeweils zwischen zwei beliebigen benachbarten Einheiten der mindestens zwei unabhängig aktivierbaren Einheiten des Sensorelements vorliegen. Der Abstand wird jeweils vom äußersten Rand der zwei unabhängig aktivierbaren Einheiten des Sensorelements gemessen. Zwischen zwei benachbarten Einheiten der mindestens zwei unabhängig aktivierbaren Einheiten des Sensorelements kann kein weiteres Sensorelement vorliegen. Zwischen zwei benachbarten Einheiten der mindestens zwei unabhängig aktivierbaren Einheiten des Sensorelements können weitere Einheiten des Behandlungselements vorliegen.

Zwischen zwei benachbarten Einheiten der mindestens zwei unabhängig aktivierbaren Einheiten des Behandlungselements kann jeweils ein Abstand zwischen 0,2 mm und 10,0 mm, bevorzugt zwischen 0,5 mm und 9,0 mm, besonders bevorzugt zwischen 1,0 mm und 8,0 mm vorhanden sein. Nach einer weiteren vorteilhaften Ausführungsform beträgt der Abstand zwischen 2,0 mm und 7,0 mm, bevorzugt zwischen 3,0 mm und 7,0 mm, besonders bevorzugt 5,0 mm. Der Abstand zwei benachbarten Einheiten der mindestens zwei unabhängig aktivierbaren Einheiten des Sensorelements kann in einer anderen bevorzugten Ausführungsform jeweils zwischen 50 % und 150 % des maximalen Durchmessers einer unabhängig aktivierbaren Einheit des Behandlungselements betragen.

Der Abstand kann zwischen zwei beliebigen benachbarten Einheiten der mindestens zwei unabhängig aktivierbaren Einheiten des Behandlungselements vorliegen. Der Abstand wird jeweils vom äußersten Rand der zwei unabhängig aktivierbaren Einheiten des Behandlungselements gemessen. Zwischen zwei benachbarten Einheiten der mindestens zwei unabhängig aktivierbaren Einheiten des Behandlungselements kann kein weiteres Behandlungselement vorliegen. Zwischen zwei benachbarten Einheiten der mindestens zwei unabhängig aktivierbaren Einheiten des Behandlungselements können weitere Einheiten des Sensorelements vorliegen.

Beispielsweise können Einheiten des Behandlungselements, die 5 mm maximalen Durchmesser haben, einen Abstand von 5 mm oder 100% des maximalen Durchmessers zur nächsten Einheit des Behandlungselements haben. Versetzt dazu sind können die Einheiten des Sensorelements in der gleichen Ebene wie die Einheiten des Behandlungselements angeordnet sein. Die Einheiten des Sensorelements, die 5 mm maximalen Durchmesser haben, können einen Abstand von 5 mm oder 100 % des maximalen Durchmessers zur nächsten Einheit des Sensorelements haben.

Die unabhängig aktivierbaren Einheiten des Sensorelements können eingerichtet sein, Messdaten betreffend einer oder mehrerer Eigenschaften eines Haut- oder Wundabschnitts innerhalb eines ersten Zeitraums aufzunehmen und Messdaten betreffend einer oder mehrerer Eigenschaften desselben Haut- oder Wundabschnitts innerhalb eines zweiten Zeitraums aufzunehmen. Auf Grundlage der ersten Messung und der zweiten Messung können die eine oder mehreren Eigenschaften der Wunde ermittelbar sein. Ein Beispiel für eine Eigenschaft der Wunde wäre, dass die Wundheilung stagniert.

Die unabhängig aktivierbaren Einheiten des Sensorelements können eingerichtet sein, Messdaten betreffend einer oder mehrerer Eigenschaften eines Haut- oder Wundabschnitts für eine Vielzahl von Zeiträumen aufzunehmen. Auf Grundlage der Vielzahl von Messungen kann die eine oder mehreren Eigenschaften der Wunde ermittelbar sein. Die Anzahl der Messungen beträgt beispielsweise drei, bevorzugt zumindest fünf, besonders bevorzugt zumindest 10, insbesondere zumindest 20.

Zwischen zwei Zeiträumen kann ein zeitlicher Abstand von zumindest 5 min, bevorzugt zumindest 15 min, bevorzugter zumindest 1 h, bevorzugter zumindest 1 d (ein Tag), liegen. Das kann für alle hierin offenbarten Zeiträume gelten. Durch mehrere Messungen über einen langen Zeitraum kann eine Entwicklung der einen oder mehreren Eigenschaften der Wunde besonders gut überwacht werden.

Die eine oder mehreren zu charakterisierenden Eigenschaften der Wunde können eine Fläche der Wunde, eine Tiefe der Wunde, ein Volumen der Wunde, ein Heilungsfortschritt der Wunde, eine Heilungskomplikation der Wunde, eine Zustandsänderung der Wunde, eine Dichte der Wunde (ermittelbar beispielsweise durch Ultraschall), ein Wassergehalt der Wunde, eine Zusammensetzung der Wunde, Gewebeschichten der Wunde und/oder ein mikrobieller Zustand der Wunde sein. Bevorzugt ist die zu charakterisierende Eigenschaft der Wunde ein mikrobieller Zustand der Wunde (d.h. die Intensität einer mikrobiellen Besiedelung der Wunde und/oder der Wundoberfläche) oder eine Zustandsänderung der Wunde. Die Überwachung der Wunde erlaubt es beispielsweise eine Veränderung des Wundraumes oder eine Veränderung der mikrobiellen Besiedelung während einer Behandlung zu ermitteln.

In einer bevorzugten Ausführungsform können die in der Wundauflage vorhandenen Bauteile eingerichtet sein, innerhalb eines Zeitraums zeitlich versetzt als Einheiten des unabhängig aktivierbaren Sensorelements oder als Einheiten des unabhängig aktivierbaren Behandlungselements zu wirken. Beispielsweise können piezoelektrischen Bauteile eingerichtet sein, innerhalb eines Zeitraums zeitlich versetzt Ultraschall zu erzeugen und Ultraschall zu empfangen. Die piezoelektrischen Bauteile können also eingerichtet sein, als Sender und als Empfänger zu agieren. Der für die Ultraschallbehandlung von einem piezoelektrischen Bauteil erzeugte Ultraschall wird in dieser Ausführungsform für die Messung verwendet, indem zeitversetzt eine Reflexion des, von demselben piezoelektrischen Bauteil emittierten, Ultraschalls gemessen wird.

Die unabhängig aktivierbaren Einheiten des Behandlungselements können so steuerbar sein, dass zu einem Zeitpunkt nur höchstens einige der Einheiten des unabhängig aktivierbaren Behandlungselements, bevorzugt höchstens 50 % der unabhängig aktivierbaren Einheiten des Behandlungselements, bevorzugter höchstens 10% der Einheiten des unabhängig aktivierbaren Behandlungselements, besonders bevorzugt höchstens eines der unabhängig aktivierbaren Einheiten des Behandlungselements aktiviert wird.

Bevorzugt ist die Anzahl der zu einem Zeitpunkt aktivierten Einheiten des Behandlungselements auf die eine oder mehrere Eigenschaften der Wunde abgestimmt. Beispielsweise wird das Behandlungselement über der gesamten Fläche der Wunde oder über einen Bereich der Wunde aktiviert, nicht aber über den die Wunde umgebenden, unversehrten Hautbereichen. Falls die Aktivierung lediglich über einen Bereich der Wundoberfläche erfolgt, so könnte dieser Bereich derjenige sein, welcher aufgrund der durch das Sensorelement aufgenommen Messdaten als gesund eingeschätzt wurde. Zur teilweisen Aktivierung des Behandlungselements werden nur bestimmte Einheiten des Behandlungselements aktiviert. Die aktivierten Einheiten des Behandlungselements können die Einheiten sein, die zu bevorzugt direkt über der Wundoberfläche liegen. Alternativ können auch die Einheiten des Behandlungselements aktiviert werden, die nur teilweise über der Wundoberfläche liegen. Die unabhängig aktivierbaren Einheiten des Behandlungselements können so steuerbar sein, dass Teilmengen der unabhängig aktivierbaren Einheiten des Behandlungselements zu unterschiedlichen Zeitpunkten für die Behandlung der Wunde aktiviert werden. Beispielsweise können zu einem ersten Zeitpunkt eine erste Teilmenge der unabhängig aktivierbaren Einheiten des Behandlungselements für die Behandlung der Wunde aktiviert werden und zu einem zweiten Zeitpunkt eine zweite Teilmenge der unabhängig aktivierbaren Einheiten des Behandlungselements für die Behandlung der Wunde aktiviert werden.

Die Wundauflage kann eine wundheilungsfördernde Substanz umfassen, die an die Wunde abgebbar ist. Die Substanz kann in die Wunde einbringbar sein und durch die Behandlung kann eine Aufnahme der Substanz in eine Zelle der Wunde erhöht oder ermöglicht werden. Beispielsweise kann die Substanz aus der Wundauflage in die Wunde diffundieren.

Die Substanz kann in einen Träger eingebracht sein. Der Träger kann die Substanz umgeben und/oder stabilisieren.

Die Wundauflage kann ein Steuerungsmittel umfassen. Das Steuerungsmittel kann eingerichtet sein, das Sensorelement und das Behandlungselement zu steuern. Insbesondere ist das Steuerungsmittel eingerichtet einzelne Einheiten der unabhängig aktivierbaren Einheiten des Sensorelements und einzelne Einheiten die unabhängig aktivierbaren Einheiten des Behandlungselements zu steuern. Das Steuerungsmittel kann ein Mikrocontroller sein.

Die Wundauflage kann ein erstes Senderempfängerelement umfassen, welches Daten an ein zweites Senderempfängerelement übertragen und von diesem empfangen kann, wobei das zweite Senderempfängerelement eine Komponente eines nicht in der Wundauflage enthaltenen Auswertungsmittels ist. Die Datenübertragung kann mittels einer Antenne realisiert sein und eingerichtet sein, Daten kontaktlos zu übertragen und/oder zu empfangen. Die kontaktlose Datenübertragung kann Daten über NFC (Near Field Communication), Bluetooth, Bluetooth Low Energy, ZigBee, WLAN (Wireless Local Area Network) und/oder ein Mobilfunknetz senden.

Das erste Senderempfängerelement kann einen Mikroprozessor umfassen.

Die Wundauflage kann eine Energieversorgungseinrichtung umfassen. Die Energieversorgungseinrichtung kann eingerichtet sein, Energie für die Wundauflage bereitzustellen.

Die Energieversorgungseinrichtung kann einen Energiespeicher umfassen. Durch die Energieversorgungseinrichtung kann gespeicherte Energie für die Wundauflage bereitstellbar sein. Die Energieversorgungseinrichtung kann eine Batterie oder einen Akku (Akkumulator) umfassen. Die Batterie kann flexibel oder nachgiebig sein. Bevorzugt ist die Batterie eine Dünnschicht-Batterie. Der Akku kann flexibel oder nachgiebig sein. Bevorzugt ist der Akku ein Dünnschicht-Akku.

Alternativ oder zusätzlich kann die Energieversorgungseinrichtung eingerichtet sein, Energie kontaktbehaftet oder kontaktlos zu empfangen. Die Energieversorgungseinrichtung kann einen Flachstecker umfassen. Über den Flachstecker kann die Energieversorgungseinrichtung mit einer externen Energiequelle, beispielsweise mit einer Steckdose, verbunden werden. Insbesondere ist die Energieversorgungseinrichtung eingerichtet, Energie durch elektromagnetische Strahlung zu empfangen. Die Energieversorgungseinrichtung kann eingerichtet sein, Energie über NFC (Near Field Communication), Bluetooth, Bluetooth Low Energy, ZigBee, WLAN (Wireless Local Area Network) und/oder ein Mobilfunknetz zu empfangen.

In einer bevorzugten Ausführungsform kann zumindest eines der unabhängig aktivierbaren Einheiten des Sensorelements über der Wunde vorhanden sein und zumindest eines der unabhängig aktivierbaren Einheiten des Sensorelements kann über der die Wunde umgebenden unversehrten Haut vorhanden sein. Das ist dann der Fall, wenn die Wundauflage auf dem die Wunde umgebenden Hautabschnitt fixiert ist. Dadurch kann sowohl eine Messung von Eigenschaften der die Wunde umgebenden gesunden bzw. intakten Haut des Patienten als auch eine Messung von Eigenschaften der Wunde durchgeführt werden. Die Daten der Messung der Eigenschaften der gesunden Haut kann als Referenz dienen und mit Daten der Messung der Wunde verglichen werden, um die eine oder mehreren Eigenschaften der Wunde zu bestimmen. Intakte oder gesunde Haut kann insbesondere ein die Wunde umgebender Hautabschnitt sein. Die eine oder mehreren Eigenschaften der Wunde können jedoch nach einer alternativen Ausführungsform ebenso ohne Referenz, d.h. ohne eine Bestimmung der Eigenschaften der gesunden Haut erfolgen.

In einer weiteren alternativen Ausführungsform muss mindestens eines der unabhängig aktivierbaren Einheiten des Sensorelements die Wunde überlagern. Dadurch kann eine Charakterisierung der Wunde durchgeführt werden. Die Daten der Messung der Wunde können mit Referenzdaten verglichen werden, um die eine oder mehreren Eigenschaften der Wunde zu bestimmen. Die Referenzdaten können das Ergebnis vergangener Messungen bei demselben Patienten sein. Alternativ können die Referenzdaten schon klassifizierte Daten sein, die in einer Datenbank abgelegt wurden. Auch kann die Klassifizierung zwischen gesunder Haut und Wunde mittels eins trainierten Modells vorgenommen werden.

Die Wundauflage kommuniziert mit einem Auswertungsmittel. Das Auswertungsmittel umfasst ein zweites Senderempfängerelement. Das zweite Senderempfängerelement ist dazu ausgestaltet, Daten von dem ersten Senderempfängerelement zu empfangen und an das erste Senderempfängerelement zu senden. Das erste Senderempfängerelement ist eine Komponente der Wundauflage. Das Auswertungsmittel umfasst mindestens einen Prozessor. Der mindestens eine Prozessor ist dazu konfiguriert ein Verfahren, das folgende Schritte umfasst, auszuführen:
a. Eine Aufnahme eines Datensatzes wird mittels des Sensorelements ausgelöst. Der Datensatz umfasst mindestens zwei Datenpunkte und jeder Datenpunkt umfasst mindestens einen Messwert einer Einheit des Sensorelements und mindestens eine Ortskoordinate, die mit der Position der Einheit des Sensorelements korrespondiert.
b. Der Datensatz wird ausgewertet, so dass mindestens eine Eigenschaft der Wunde ermittelt wird.
c. Auf Basis der in Schritt b. ermittelten mindestens einen Eigenschaft der Wunde werden die mindestens zwei unabhängig aktivierbaren Einheiten des Behandlungselements aktiviert oder selektiv aktiviert.

Der Datensatz umfasst mindestens eine Ortskoordinate, die eine Zuordnung jedes Messwertes relativ zu einem Punkt auf der Fläche der Wundauflage erlauben. Bevorzugt wird ein kartesisches Koordinatensystem in zwei Dimensionen verwendet. Eine dritte Dimension kann eingeführt werden, um beispielsweise die Tiefe eines Wundabschnitts relativ zu einem intakten Hautabschnitt zu beschreiben.

Der mindestens eine Messwert kann beispielsweise mindestens einen Spannungswert und/oder mindestens einen Stromwert und/oder mindestens einen Helligkeitswert und/oder einen Widerstand umfassen.

In einer bevorzugten Ausführungsform umfasst jeder Datenpunkt des Datensatzes einen Zeitstempel bzw. eine Zeitangabe, welcher angibt zu welchem Zeitpunkt, der mindestens eine Messwert aufgenommen wurde. Der Zeitstempel kann beispielsweise eine Uhrzeit mit Datum oder einen Wert, der die seit Beginn der Messung bzw. Wechsel der Wundauflage verstrichenen Zeiteinheiten, beispielsweise Sekunden, repräsentiert, enthalten.

Der Datensatz wird von dem Auswertungsmittel ausgewertet und die mindestens eine Eigenschaft der Wunde wird ermittelt.

Unterschiedliche Bereiche der Wundoberfläche können unterschiedliche Eigenschaften aufweisen. In einer bevorzugten Ausführungsform können unterschiedliche Bereiche der Wundoberfläche klassifiziert und darüber hinaus intakte Haut von Wundoberfläche unterschieden werden. Durch die Unterscheidung zwischen Wundfläche und intakter Haut könnten die Wundfläche und die Wundränder bestimmt werden. Die Sensoreinheit kann beispielsweise auf Basis photometrischer Messungen in Wunde vorkommendes nekrotisches Gewebe, entzündetes Gewebe oder Granulationsgewebe identifizieren. Hierbei können bekannte Verfahren der Bildauswertung und Mustererkennung, auch unter Einsatz von Trainingsdaten, angewandt werden.

Auf Basis der von dem Sensorelement unter Einsatz einer Bildauswertung ermittelten Eigenschaften der Wunde wird das Behandlungselement aktiviert. Bevorzugt wird das Behandlungselement nur über der Wundfläche aktiviert. Ebenfalls bevorzugt wird das Behandlungselement nur über ausgewählten Anteilen der Wundoberfläche aktiviert, wobei die Aktivierung bevorzugt über solchen Anteilen der Wundoberfläche erfolgt, bei denen eine Störung der Wundheilung vorliegt bzw. vorzuliegen scheint. Die unabhängig aktivierbaren Einheiten des Behandlungselements erlauben somit eine ortsspezifische Behandlung der Wunde. Wundbereiche, bei denen nach Auswertung der Messdaten keine Störung der Wundheilung vorliegt bzw. vorzuliegen scheint, werden nicht behandelt. Diese Bereiche werden daher von einer unnötigen Behandlung verschont.

Die Dauer der Aktivierung des Behandlungselements kann mittels eines voreingestellten Schemas und unabhängig von den während der Behandlung ermittelten Eigenschaften der Wunde festgelegt werden. Diese Festlegung kann beispielsweise bereits werkseitig durch den Hersteller der Wundauflage erfolgen oder einstellbar durch einen Behandler. In einer anderen Ausführungsform kann die Dauer der Aktivierung des Behandlungselements von den während der Behandlung ermittelten Eigenschaften der Wunde abhängen. Wird beispielsweise in der Wunde ein Biofilm erkannt, so werden die Einheiten des Behandlungselements über dem Biofilm aktiviert. Bei den Einheiten des Behandlungselements kann es sich um piezoelektrische Ultraschallsensoren handeln, die den Biofilm durch Erzeugen von Kavitation zerstören können.

In einer bevorzugten Ausführungsform werden die Schritte a, b und c des oben beschriebenen Verfahrens mehrmals ausgeführt, bis eine sogenannte Abbruchbedingung erfüllt ist. Nachdem die Wundauflage auf die Wundfläche aufgebracht wurde, kann der Benutzer das Verfahren starten oder das Verfahren wird automatisch gestartet. Startet der Benutzer das Verfahren, so kann dies beispielsweise über eine Eingabe mittels des Auswertungsmittels geschehen. Das Verfahren durchläuft nach Start des Verfahrens in einer Schleife mehrmals die Schritte a, b und c. Eine mögliche Abbruchbedingung kann beispielsweise die vollständige Heilung der Wunde sein. Eine weitere denkbare Abbruchbedingung könnte beispielsweise das Erreichen einer vorgegebenen Anzahl von Schleifendurchläufen sein. Eine weitere mögliche Abbruchbedingung kann eine manuelle Eingabe eines Benutzers sein. Beispielsweise kann der Benutzer mittels des Auswertungsmittels einen Befehl zum Abbruch der aktuellen Verfahrensschleife eingeben. Der Benutzer kann auch durch das Entfernen der Wundauflage vom Körper eine Abbruchbedingung erfüllen. In einer besonders bevorzugten Ausführungsform kann die Abbruchbedingung das Erreichen einer vorbestimmten Zeitdauer oder den Wechsel der Wundauflage umfassen.

In einer bevorzugten Ausführungsform umfasst das Verfahren mehr als eine Abbruchbedingungen. Die Abbruchbedingung, die zuerst erfüllt ist, beendet das Verfahren. Beispielsweise kann das Entfernen der Wundauflage eine erste Abbruchbedingung sein und eine Zeitdauer eine zweite Abbruchbedingung sein. Wird die Wundauflage entfernt, bevor die Zeitdauer erreicht ist, so wird das Verfahren abgebrochen.

In einer weiteren Ausführungsform ist zwischen den Verfahrensschleifen ein zeitlicher Abstand vorgesehen. Bevorzugt ist der zeitliche Abstand konstant. Der zeitliche Abstand kann bevorzugt auch von den Eigenschaften der Wunde abhängen. Wird beispielsweise festgestellt, dass der Zustand der Wunde sich verschlechtert hat, so werden kleinere zeitliche Abstände zwischen zwei Verfahrensschleifen gewählt.

In einer bevorzugten Ausführungsform wird die ordnungsgemäße Aufnahme der Daten überwacht. Sind die Abstände zwischen den Verfahrensschleifen und die Dauer der einzelnen Verfahrensschritte mittels des Prozessors vorbestimmt, so ist mit einer bestimmten Anzahl von Datenpunkten zu rechnen. Fehlen Datenpunkte, beispielsweise aufgrund eines Abbruchs der Verbindung zwischen dem erstem Senderempfängerelement und dem zweiten Senderempfängerelement, so kann ein Warnsignal ausgegeben werden. In einer anderen Ausführungsform kann ein Zähler für Fehler implementiert werden. Ab einer bestimmten Anzahl von Fehlern kann die Messung als fehlerhaft gekennzeichnet werden.

Gemäß einer gleichfalls bevorzugten Ausführungsform kommunizieren mehrere Wundauflagen mit einem einzigen Auswertungsmittel. Jede Wundauflage besitzt dann eine eindeutige Kennzeichnung, beispielsweise eine Geräte-ID. Die eindeutige Kennzeichnung wird zusammen mit den durch das Sensorelement aufgenommen Daten an das Auswertungsmittel geschickt. Damit wird ein eine eindeutige Zuordnung eines bestimmten Datensatzes zu einer Wundauflage möglich. Zur leichteren Benutzung ist eine Zuweisung der eindeutigen Kennzeichnung zu einer benutzerfreundlichen Kennzeichnung, wie sie beispielsweise ein Patientenname oder eine Bettnummer darstellen könnte, möglich.

Das Auswertungsmittel kann von der Wundauflage beabstandet sein. Beispielsweise kann das Auswertungsmittel ein Server sein, welcher sich in einem anderen Raum als der Patient befindet. Das Auswertungsmittel kann Daten der Messung erhalten oder empfangen und auf Grundlage der Daten die eine oder mehreren Eigenschaften der Wunde ermitteln. Das Auswertungsmittel umfasst mindestens einen Prozessor, der eingerichtet sein kann, auf Grundlage der von der Sensoreinheit durchgeführten Messungen die eine oder mehreren Eigenschaften der Wunde zu ermitteln. In einer bevorzugten Ausführungsform wird die Wunde in Segmente unterteilt und jedem Segment wird eine Eigenschaft der Wunde zugeordnet. Das Auswertungsmittel kann eine Datenbank umfassen. Die Datenbank kann gespeicherte Messungen umfassen. Zur Ermittlung der einen oder mehreren Eigenschaften der Wunde kann das Auswertungsmittel eine Messung mit den gespeicherten Daten vorhergehender Messungen an demselben oder an unterschiedlichen Patienten vergleichen.

In einer bevorzugten Ausführungsform umfasst das Auswertungsmittel ein Ausgabemittel oder ist mit einem Ausgabemittel verbunden. Das Ausgabemittel kann ein optisches oder akustisches Signal erzeugen. Bevorzugt ist das Ausgabemittel ein Bildschirm.

Zu jeder Aufnahme des Datensatzes wird die aktuelle Zeit aufgenommen und die mindestens eine Eigenschaft der Wunde im Zeitverlauf auf dem Ausgabemittel dargestellt.

In einer bevorzugten Ausführungsform wird die mindestens eine Eigenschaft der Wunde, die zu einem ersten Zeitpunkt bestimmt wird, gespeichert und mit der mindestens einen Eigenschaft der Wunde, die zu einem zweiten Zeitpunkt bestimmt wird, verglichen. Mittels eines Vergleichs der mindestens einen Eigenschaft der Wunde zum ersten Zeitpunkt und der mindestens einen Eigenschaft der Wunde zum zweiten Zeitpunkt kann der Heilungsprozess der Wunde überwacht werden. Der Vergleich der mindestens einen Eigenschaft der Wunde zum ersten Zeitpunkt und der mindestens einen Eigenschaft der Wunde zum zweiten Zeitpunkt kann beispielsweise ergeben, dass eine Veränderung der Wundfläche aufgetreten ist.

In einer bevorzugten Ausführungsform umfasst das Auswertungsmittel ein Eingabemittel. Das Eingabemittel ist bevorzugt eine Tastatur und/oder eine Maus, alternativ bevorzugt ist das Eingabemittel ein Touchscreen. Das Eingabemittel kann unter anderem dazu dienen, dass der Behandelnde einen Befehl zum Abbruch der aktuellen Verfahrensschleife einleiten kann.

### FIGUREN

Fig. 1 zeigt schematische eine Ausführungsform der Wundauflage mit absorbierender Zwischenschicht und Wundkontaktschicht.
Fig. 2 zeigt schematisch eine mögliche Ausführungsform des Behandlungselements in einer Ebene.
Fig. 3a zeigt schematisch eine mögliche Ausführungsform umfassend mehrere Anordnungen, die aus 2 unabhängig aktivierbaren Einheiten des Sensorelements und 2 unabhängig aktivierbaren Einheiten des Behandlungselements bestehen.
Fig. 3b zeigt schematisch, wie die mögliche Ausführungsform durch das Aneinanderfügen von Anordnungen aufgebaut ist.
Fig. 4a zeigt schematisch eine andere mögliche Ausführungsform, in der die Anordnungen, die aus 2 unabhängig aktivierbaren Einheiten des Sensorelements und aus 2 unabhängig aktivierbaren Einheiten des Behandlungselements bestehen. Es werden zwei verschiedene Typen von Bauteilen für die Einheiten des Sensorelements verwendet.
Fig. 4b zeigt schematisch, wie die Ausführungsformdurch das Aneinanderfügen von Anordnungen aufgebaut ist.
Fig. 5 zeigt schematisch eine weitere mögliche Ausführungsform, in der die Anordnungen, die aus 8 unabhängig aktivierbaren Einheiten des Sensorelements und einer unabhängig aktivierbaren Einheit des Behandlungselements bestehen.
Fig. 6 zeigt schematisch eine mögliche Ausführungsform des Sensorelements und des Behandlungselements in 3 Dimensionen.
Fig. 7 zeigt eine schematische Darstellung einer Ausführungsform des Auswertungsmittels.
Fig. 8 zeigt eine schematische Darstellung einer Ausführungsform des Verfahrens.

### FIGURENBESCHREIBUNG

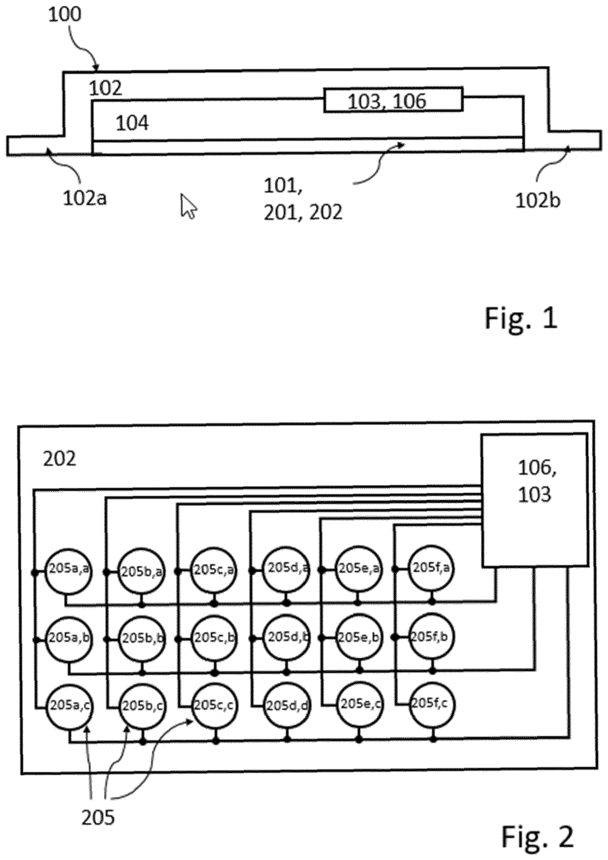

Fig. 1 zeigt eine mögliche Ausführungsform der Wundauflage 100 die in einem örtlich beschränkten Bereich einer Wundstelle einen therapeutischen Effekt ausüben kann. Die Wundauflage 100 umfasst eine Wundkontaktschicht 101 mit Sensorelement 201 und Behandlungselement 202, eine Abdeckschicht 102 und einer absorbierenden Zwischenschicht 104, wobei die Zwischenschicht 104 zwischen Wundkontaktschicht 101 und Abdeckschicht 102 angeordnet ist. Die Wundkontaktschicht 101 liegt direkt auf der Haut oder der Wundstelle des Patienten auf. Die Abdeckschicht 102 kann adhäsive Bereiche 102a, 102b auf der dem Patienten zugewandten Seite der Abdeckschicht aufweisen. Die adhäsive Bereiche 102a, 102b dienen der Befestigung der Wundauflage 100 am Patienten.

Die Wundauflage 100 umfasst darüber hinaus ein erstes Senderempfängerelement 103 und eine Stromquelle 106. Die Stromquelle 106 versorgt das Behandlungselement 104 und das erste Senderempfängerelement 103 mit Energie. Bei der Stromquelle 106 kann es sich um eine Batterie handeln. Alternativ kann die Stromquelle 106 Energie mittels Energy Harvesting bereitstellen.

Fig. 2 zeigt eine mögliche Ausführungsform des Behandlungselements 202 der Wundauflage 100. In dieser Ausführungsform sind alle unabhängig aktivierbaren Einheiten 205 des Behandlungselements 202 in einer einzigen Ebene parallel zur Wundoberfläche angeordnet. Die einzelnen unabhängig aktivierbaren Einheiten 205 des Behandlungselements können auf einem flexiblen Substrat, z.B. Silikon, aufgebracht oder eingebettet sind.

Die unabhängig aktivierbaren Einheiten 205a,a bis 205f,c sind so geschaltet, dass sie einzeln aktiviert werden können.

Mittels der Stromquelle 106 werden die unabhängig aktivierbaren Einheiten 205a,a bis 205f,c mit Energie versorgt. Das erste Senderempfängerelement 103 ist ebenfalls elektronisch an die Stromquelle 106 gekoppelt. Mittels des ersten Senderempfängerelements 103 werden Daten, die den Zustand der Wunde betreffen an ein zweites Senderempfängerelement (in Fig. 2 nicht dargestellt) gesendet. Ebenso kann das erste Senderempfängerelement 103 Anweisungen zur Aktivierung der unabhängig aktivierbaren Einheiten 205 über das zweite Senderempfängerelement empfangen.

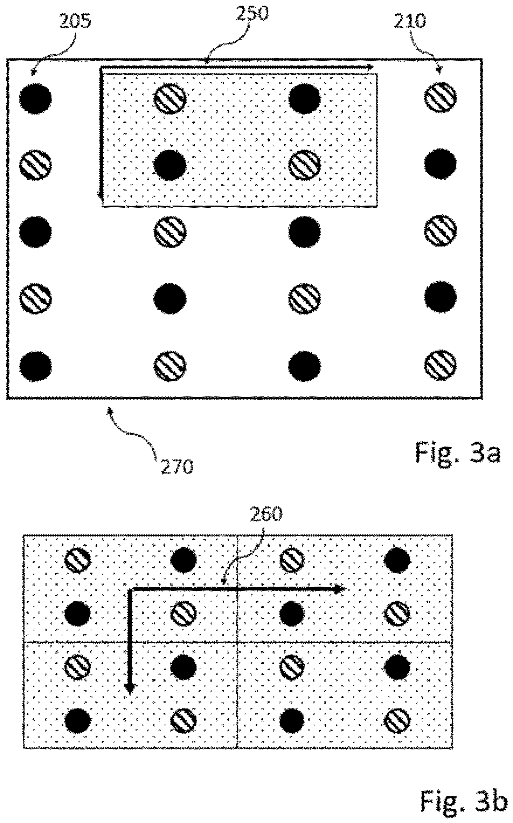

Fig. 3a zeigt eine beispielhafte eine mögliche Ausführungsform von Anordnungen, die wiederholt, lückenlos und mittels translationssymmetrischer Operationen aneinandergefügt wurden, 270 in zwei Dimensionen. Die unabhängig aktivierbaren Einheiten 205 des Behandlungselements und die unabhängig aktivierbaren Einheiten 210 des Sensorelements sind abwechselnd angeordnet. Bei den Einheiten 205 des Behandlungselements kann es sich um piezoelektrische Ultraschallemitter handeln und die Einheiten 210 des Sensorelements können piezoelektrische Ultraschallsensoren sein. Die Anordnung 250 besteht in diesem Beispiel aus zwei unabhängig aktivierbaren Einheiten 205 des Behandlungselements und zwei unabhängig aktivierbaren Einheiten 210 des Sensorelements. Das Volumen, das jede der Anordnungen 250 einnimmt, ist in diesem Fall ein Rechteck, das einen Spezialfall des Parallelogramms darstellt. Durch translationssymmetrische Operationen 260, dargestellt in Fig. 3b, werden die Anordnungen 250 aneinandergefügt.

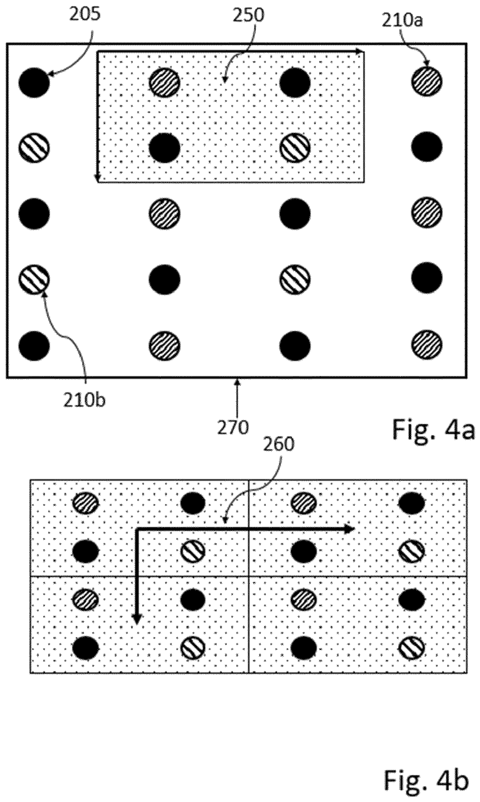

Fig.4a zeigt eine andere mögliche Ausführungsform von Anordnungen, die wiederholt, lückenlos und mittels translationssymmetrischer Operationen aneinandergefügt wurden, 270 bestehend aus Anordnungen 250 mit je zwei gleichen Einheiten 205 des Behandlungselements und je zwei verschiedenen Einheiten 210 des Sensorelements. Bei den Einheiten 205 des Behandlungselements kann es sich um Fotodioden handeln und die Einheiten 210 des Sensorelements können Fotosensoren 210a und piezoelektrische Ultraschallsensoren 210b sein. In Fig. 4b wird gezeigt, wie die Anordnungen 250 aneinandergefügt werden.

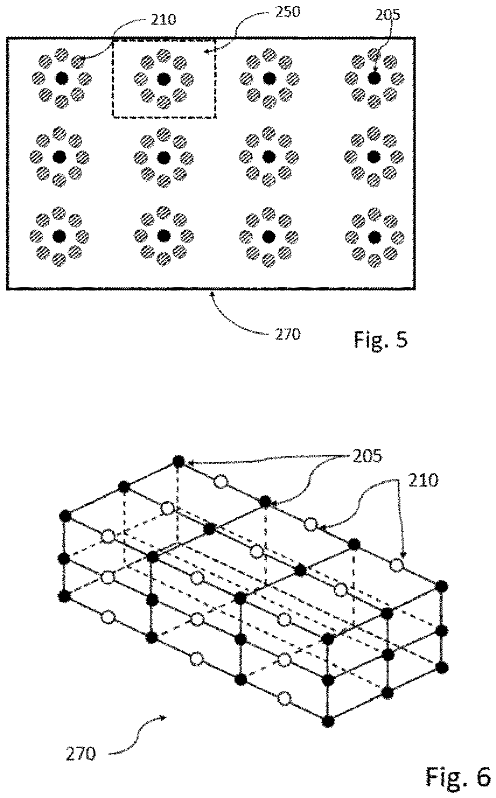

Fig. 5 zeigt eine weitere mögliche Ausführungsform von Anordnungen, die wiederholt, lückenlos und mittels translationssymmetrischer Operationen aneinandergefügt wurden, 270 in zwei Dimensionen. Die Anordnungen 250besteht aus einer unabhängig aktivierbaren Einheit 205 des Behandlungselements und acht unabhängig aktivierbaren Einheiten 210 des Sensorelements. Eine Vielzahl von unabhängig aktivierbaren Einheiten 210 des Sensorelements kann dann von Vorteil sein, wenn eine höhere Auflösung der Wunde oder das genaue Ermitteln mehrerer Wundeigenschaften ermöglicht werden soll. Denkbar ist eine Vielzahl an Fotosensoren und nur vergleichsweise wenigen Fotodioden.

In Fig. 6 ist eine einfache beispielhafte Ausführungsform 270 von Anordnungen dargestellt, die wiederholt, lückenlos und mittels translationssymmetrischer Operationen in 3 Dimensionen aneinandergefügt wurden. Eine Anordnung 250 umfasst je eine unabhängig aktivierbare Einheit 205 des Behandlungselements und eine unabhängig aktivierbare Einheit 210 des Sensorelements. Das Volumen, das jede der Anordnungen einnimmt, kann ein Quader sein. Der Quader ist ein Parallelepiped.

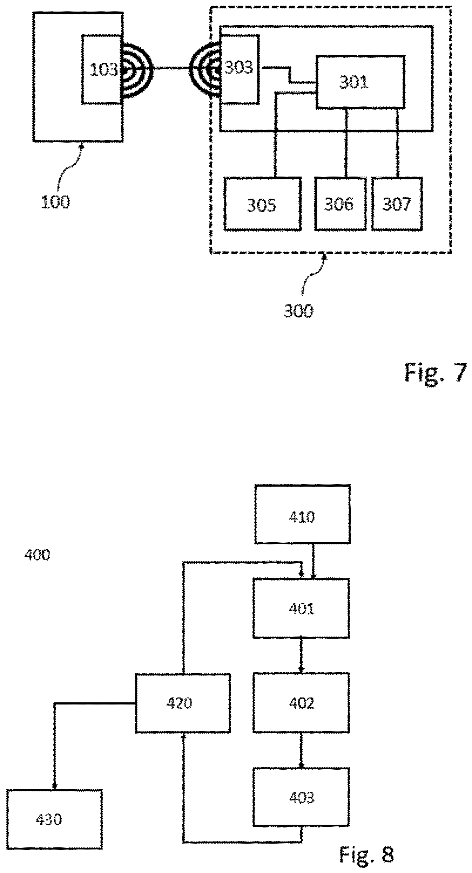

In Fig. 7 ist Auswertungsmittel 300 und seine Interaktion mit der Wundauflage 100 schematisch dargestellt. Die erste Senderempfängerelement 103 kommuniziert mit dem zweiten Senderempfängerelement 303. Dadurch können Sensordaten, die von der Wundauflage aufgenommen wurden, an das Auswertungsmittel 300 gesendet werden und Aktivierungsanweisungen für das Behandlungselement an die Wundauflage 100 übermittelt werden. Das Auswertungsmittel 100 umfasst darüber einen Prozessor 301. Der Prozessor 301 wertet die mittels des zweiten Senderempfängerelements 303 empfangenen Daten aus und erstellt eine Aktivierungsanweisung. Die Aktivierungsanweisung kontrolliert, welche Einheiten 205 des Behandlungselements 202 zur Behandlung aktiviert werden. Zur Erstellung dieser Aktivierungsanweisung kann eine Datenbank 305 mit dem Prozessor 301 verbunden sein. Die Datenbank 305 kann die Daten, die von der Wundauflage 100 aufgenommen wurden, zudem die auf Basis der durchgeführten Messungen bestimmten Wundeigenschaften sowie die von dem Prozessor 301 erstellten Aktivierungsanweisungen speichern. Weitere Bestandteile des Auswertungsmittel 300 sind ein Ausgabemittel 306 und ein Eingabemittel 307. Das Ausgabemittel 306 und das Eingabemittel 307 kann beispielsweise ein Touchscreen sein. Fig. 8 zeigt eine schematische Darstellung des Verfahrens 400, wobei auf die in Fig. 7 gezeigten Komponenten Bezug genommen wird. Das Verfahren wird gestartet (Schritt 410) durch Aufbringen der Wundauflage oder Benutzereingabe über das Eingabemittel 307. Das Auswertungsmittel 300 sendet den Befehl zur Aufnahme eines Datensatzes an die Wundauflage 100. Die Einheiten des Sensorelements nehmen je einen Sensorwert auf in Schritt 401. Der Sensorwert wird mit der entsprechenden, zweidimensionalen Ortskoordinate auf der Wundauflage 100 der Einheit des Sensorelements verbunden. Der Datensatz enthält außerdem einen Zeitstempel und eindeutige Kennzeichnung der Wundauflage, von der die Daten gesendet wurden. In Schritt 402 werden die an das Auswertungsmittel 300 gesendeten Daten ausgewertet und die Eigenschaften der Wunde bestimmt. Sind die Einheiten 210 des Sensorelements Fotosensoren, so kann über den Farbgradient zwischen zwei benachbarten Einheiten 210 des Sensorelements die Fläche der Wunde bestimmt werden. Der Prozessor 301 bestimmt dann in Schritt 403 die Einheiten 205 des Behandlungselements, die über der vorab ermittelten Wundfläche liegen. Der Prozessor 301 sendet eine Aktivierungsanweisung mittels des zweiten Senderempfängerelements 303 an das erste Senderempfängerelement 103. Die Wundauflage 100 steuert die in der Aktivierungsanweisung vorgegeben Einheiten 205 des Behandlungselements für eine vorgegebene Zeitdauer an. Danach wird überprüft, ob die Wundauflage 100 noch auf der Haut aufliegt. Ist die Wundauflage 100 von der Haut abgezogen worden, so erfüllt dies die Abbruchbedingung 420 und das Verfahren wird abgebrochen (Schritt 430). Ist die Abbruchbedingung 420 nicht erfüllt, so werden die Schritte 401, 402 und 403 erneut ausgeführt.

## Patentansprüche

1. Wundauflage (100), die in einem örtlich beschränkten Bereich einer Wundstelle einen therapeutischen Effekt ausüben kann, umfassend:
- eine Abdeckschicht (102), die bei Gebrauch der Wundauflage (100) wundabseitig angeordnet ist,
- ein Behandlungselement (202),
- ein Sensorelement (201),
- eine optionale Wundkontaktschicht (101), die bei Gebrauch der Wundauflage (100) wundseitig angeordnet ist,
**dadurch gekennzeichnet,**
**dass** das Behandlungselement (202) mindestens zwei unabhängig aktivierbare Einheiten umfasst, und das Sensorelement (201) mindestens zwei unabhängig aktivierbare Einheiten umfasst.

2. Wundauflage (100) nach Anspruch 1, wobei die Wundauflage (100) mindestens zwei identische Anordnungen umfasst, welche jeweils mindestens eine unabhängig aktivierbare Einheit (210) des Sensorelements (201) und mindestens eine unabhängig aktivierbare Einheit (205) des Behandlungselements (202) umfassen, und wobei die mindestens zwei Anordnungen wiederholt, lückenlos und mittels translationssymmetrischer Operationen (260) aneinandergefügt werden.

3. Wundauflage (100) nach Anspruch 1 oder 2, wobei die mindestens zwei unabhängig aktivierbaren Einheiten (210) des Sensorelements (201) in einer Ebene aus einer Menge erster Ebenen, welche parallel zu der Wundkontaktschicht (101) sind, angeordnet sind, und die mindestens zwei unabhängig aktivierbaren Einheiten (205) des Behandlungselements (202) in einer Ebene aus einer Menge zweiter Ebenen, welche parallel zu der Wundkontaktschicht (101) sind, angeordnet sind.

4. Wundauflage (100) nach Anspruch 3, wobei die Menge erster Ebenen mit der Menge zweiter Ebenen identisch ist.

5. Wundauflage (100) nach mindestens einem der vorgenannten Ansprüche, wobei die mindestens zwei unabhängig aktivierbaren Einheiten (210) des Sensorelements (201) mindestens einen der folgenden Bauteile ausgewählt aus Photosensoren, piezoelektrische Ultraschallsensoren, Impedanzsensoren oder Widerstandssensoren und die mindestens zwei unabhängig aktivierbaren Einheiten (205) des Behandlungselements (202) mindestens einen der folgenden Bauteile ausgewählt aus Photodioden, Lichtleiter, piezoelektrische Ultraschallemitter, elektrisch leitende Elemente oder Thermoelemente umfassen.

6. Wundauflage (100) nach mindestens einem der vorgenannten Ansprüche, wobei
- zwischen zwei benachbarten Einheiten der mindestens zwei unabhängig aktivierbaren Einheiten (210) des Sensorelements (201) jeweils ein Abstand zwischen 0,2 mm und 10,0 mm, bevorzugter zwischen 3,0 mm und 7,0 mm, gemessen jeweils von einem Rand einer aktivierbaren Einheit (210) des Sensorelements (201) zum Rand der benachbarten Einheit (210) des Sensorelements (201), vorliegt,
- und zwischen zwei benachbarten Einheiten der mindestens zwei unabhängig aktivierbaren Einheiten (205) des Behandlungselements (202) jeweils ein Abstand zwischen 0,2 mm und 10,0 mm, bevorzugter zwischen 3,0 mm und 7,0 mm, gemessen jeweils von einem Rand einer aktivierbaren Einheit (205) des Behandlungselements (202) zum Rand der benachbarten Einheit (205) des Behandlungselements (202), vorliegt.

7. Wundauflage (100) nach mindestens einem der vorgenannten Ansprüche, wobei
- der Abstand zwischen zwei benachbarten Einheiten der mindestens zwei unabhängig aktivierbaren Einheiten (210) des Sensorelements (201) jeweils zwischen 50 % und 150 % des maximalen Durchmessers einer unabhängig aktivierbaren Einheit (210) des Sensorelements (201) gemessen jeweils von einem Rand einer aktivierbaren Einheiten (210) des Sensorelements (201) zum Rand der benachbarten Einheit (210) des Sensorelements (201) beträgt,
- der Abstand zwischen zwei benachbarten Einheiten der mindestens zwei unabhängig aktivierbaren Einheiten (205) des Behandlungselements (202) jeweils zwischen 50 % und 150 % des maximalen Durchmessers einer unabhängig aktivierbaren Einheit (205) des Behandlungselements (202) gemessen jeweils von einem Rand einer aktivierbaren Einheit (205) des Behandlungselements (202) zum Rand der benachbarten Einheit (205) des Behandlungselements (202) beträgt.

8. Wundauflage (100) nach mindesten einem der vorgenannten Ansprüche, wobei die Wundauflage (100) ein Steuerungsmittel zur Aktivierung des Sensorelements (201) sowie des Behandlungselements (202) umfasst.

9. Wundauflage (100) nach mindesten einem der vorgenannten Ansprüche, wobei die Wundauflage (100) ein erstes Senderempfängerelement (103), welches Daten übertragen und empfangen kann umfasst.

10. Auswertungsmittel, welches mit einer Wundauflage (100) nach einem oder mehreren der Ansprüche 1 bis 9 kommuniziert, wobei das Auswertungsmittel mindestens einen Prozessor (301) umfasst.

11. Auswertungsmittel nach Anspruch 10, wobei der Prozessor (301) dazu konfiguriert ist:
- eine Aufnahme eines Datensatzes mittels des Sensorelements (201) auszulösen, wobei der Datensatz Datenpunkte umfasst, welche zu einem der mindestens zwei unabhängig aktivierbaren Einheiten (210) des Sensorelements (201) gehören, und die Datenpunkte einen Messwert und eine Ortskoordinate, die mit der Position der Einheit (210) des Sensorelements (201) korrespondiert, umfassen.
- den Datensatz auszuwerten, so dass mindestens eine Eigenschaft der Wunde ermittelt wird,
- eine Aktivierung oder selektive Aktivierung der mindestens zwei unabhängig aktivierbaren Einheiten (205) des Behandlungselements (202) auf Basis der mindestens einen ermittelten Eigenschaft der Wunde auszulösen.

12. Auswertungsmittel nach Anspruch 10 oder 11, wobei das Auswertungsmittel ein zweites Senderempfängerelement (303) umfasst, welches dazu ausgestaltet ist Daten von dem ersten Senderempfängerelement (103) zu empfangen und an das erste Senderempfängerelement (103) zu senden.

13. Verfahren zur Steuerung des in der Wundauflage (100) nach mindestens einem der Ansprüche 1 bis 9 vorhandenen Behandlungselements (202), umfassend die Schritte:
- Aufnahme eines Datensatzes mittels der mindestens zwei unabhängig aktivierbaren Einheiten (210) des Sensorelements (201), wobei der Datensatz Datenpunkte umfasst, welche zu einem der mindestens zwei unabhängig aktivierbaren Einheiten (210) des Sensorelements (201) gehören, und die Datenpunkte einen Messwert und eine Ortskoordinate, die mit der Position der Einheit (210) des Sensorelements (201) korrespondiert, umfassen,
- Auswertung des Datensatzes, so dass mindestens eine Eigenschaft der Wunde ermittelt wird,
- Aktivierung oder selektive Aktivierung der mindestens zwei unabhängig aktivierbaren Einheiten (205) des Behandlungselements (202) auf Basis der mindestens einen ermittelten Eigenschaft der Wunde,
wobei die Auswertung vorzugsweise unter Verwendnung eines Auswertungsmittel nach einem der Ansprüche 10 bis 12 erfolgt.

14. Verfahren nach Anspruch 13, wobei die Wunde in Segmente unterteilt wird und jedem Segment eine Eigenschaft der Wunde zugeordnet wird.

15. Verfahren nach Anspruch 13 oder 14, wobei die mindestens eine Eigenschaft der Wunde eine Fläche der Wunde, eine Tiefe der Wunde, eine Zustandsänderung der Wunde und/oder ein mikrobieller Zustand der Wunde ist.

16. Verfahren nach mindestens einem der Ansprüche 13 bis 15, wobei alle Schritte mindestens einmal ausgeführt werden und wobei weiterhin alle Schritte wiederholt werden, bis eine Abbruchbedingung erfüllt ist.

17. Verfahren nach Anspruch 16, wobei jeder Datenpunkt eine Zeitangabe umfasst und wobei die mindestens eine Eigenschaft der Wunde als Funktion der aufgenommenen Zeitangaben auf einem Ausgabemittel (306) dargestellt werden kann.

18. Kit umfassend die Wundauflage (100) nach mindestens einem der Ansprüche 1 bis 9 und das Auswertungsmittel nach einem der Absprüche 10 bis 12.
